(19) Europäisches Patentamt
European Patent Office
Office européen des brevets

(11) Veröffentlichungsnummer: **0 045 995**
**B1**

# (12) EUROPÄISCHE PATENTSCHRIFT

(45) Veröffentlichungstag der Patentschrift: **14.03.84**

(51) Int. Cl.³: **C 07 D 229/00** // C08G18/02

(21) Anmeldenummer: **81200891.0**

(22) Anmeldetag: **10.08.81**

## (54) Verfahren zur Herstellung eines isocyanuratfreien Uretdions aus Isophorondiisocyanat.

(30) Priorität: **13.08.80 DE 3030513**

(43) Veröffentlichungstag der Anmeldung: **17.02.82 Patentblatt 82/7**

(45) Bekanntmachung des Hinweises auf die Patenterteilung: **14.03.84 Patentblatt 84/11**

(84) Benannte Vertragsstaaten: **BE CH DE FR GB IT LI LU NL SE**

(56) Entgegenhaltungen:
**DE - A - 1 643 170**
**DE - A - 1 670 720**
**DE - A - 1 934 763**

(73) Patentinhaber: **CHEMISCHE WERKE HÜLS AG, Postfach 1320, D-4370 Marl 1 (DE)**

(72) Erfinder: **Disteldorf, Josef, Dr., Am Sengenhoff 2a, D-4690 Herne 1 (DE)**
Erfinder: **Hübel, Werner, Dr., Birnenbruchstrasse 34, D-4690 Herne 1 (DE)**
Erfinder: **Wolf, Elmar, Dr., Stauffenbergstrasse 7, D-4350 Recklinghausen (DE)**

(74) Vertreter: **Steil, Hanna, Dipl.-Chem., RSP PATENTE - PB 15 Postfach 1320, D-4370 Marl 1 (DE)**

**EP 0 045 995 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# Verfahren zur Herstellung eines isocyanuratfreien Uretdions aus Isophorondiisocyanat

Die Erfindung betrifft ein Verfahren zur Herstellung eines isocyanuratfreien Uretdions des 3-Isocyanatomethyl-3,5,5-trimethylcyclohexylisocyanats (Isophorondiisocyanat, IPDI).

Während aromatisch substituierte Uretdione und ihre Herstellung durch Dimerisierung von aromatischen Isocyanaten mit tertiären Aminen bzw. Phosphinen als Katalysatoren bereits seit langem bekannt sind, werden erstaunlicherweise aliphatisch substituierte Uretdione dagegen erst in der DE-OS 1 670 720 zum ersten Male beschrieben.

Die gemäß der Lehre der DE-OS 1 670 720 aus Isocyanaten hergestellten aliphatischen Uretdione enthalten allerdings in beträchtlichem Maß die entsprechenden Isocyanurate als »Verunreinigung« (Beispiel 1 ca. 40% Butylisocyanurat; Beispiel 2a 49% Ethylisocyanurat, 2b 59% Ethylisocyanurat, 2c 79% Ethylisocyanurat).

Auch bei der Dimerisierung des Isophorondiisocyanats entsprechend dem in der DE-OS 1 670 720 beschriebenen Verfahren wird kein reines Uretdion, sondern nur ein Gemisch von Reaktionsprodukten erhalten, das maximal aus 80% IPDI-Uretdion besteht. Der Rest ist nicht abtrennbares Isophorondiisocyanat-Isocyanurat.

Die DE-OS 1 934 763 befaßt sich ausschließlich mit der Oligomerisierung von IPDI mit tertiären Phosphinen.

Die gemäß der Lehre dieser DE-OS erhaltenen Reaktionsprodukte bestehen aus ca. 60 Gew.-T. dimeren (in der Hitze spaltbarem) und ca. 40 Gew.-T. trimerem bzw. höheroligomerisiertem (in der Hitze nicht mehr spaltbarem) IPDI. Durch geeignete Verfahrensvarianten (z. B. niedriger Umsatz) kann der Dimer-Anteil noch auf ca. 80 Gew.-T. erhöht werden. Eine weitere Erhöhung des Uretdiongehalts ist nicht mehr möglich, da der Katalysator (tert. Phosphine) nicht nur die Dimerisierung, sondern auch die Trimerisierung des IPDI zum entsprechenden Isocyanurat katalysiert.

Mit den zum Stand der Technik gehörenden Dimerisierungskatalysatoren gemäß den DE-OS 1 670 720 und 1 934 763 war es bis zum jetzigen Zeitpunkt nicht möglich, ein isocyanuratfreies Uretdion des IPDI herzustellen, d. h., es lag stets ein Gemisch von Uretdion und Isocyanurat bzw. deren höheren Oligomeren vor. Ein solches isocyanuratfreies Uretdion des IPDI ist aber von großem Interesse, da hiermit eine wirtschaftliche und gezielte Weiterreaktion mit Diolen zur Herstellung von wertvollen Ausgangsverbindungen für die Polyurethanchemie erst möglich wird.

Durch die Erfindung wurde nun ein Weg gefunden, der zu einem Uretdion des IPDI hoher Reinheit (> 98%) führt.

Gegenstand der Erfindung ist daher ein Verfahren zur Herstellung eines praktisch isocyanuratfreien Uretdions aus Isophorondiisocyanat, welches in der Hitze zu über 98% wieder in Isophorondiisocyanat rückspaltbar ist, dadurch gekennzeichnet, daß man Isophorondiisocyanat, gegebenenfalls in einem inerten organischen Lösungsmittel, mit Hilfe eines Katalysators der allgemeinen Formel

$$X_mP(NR_2)_{3-m}$$

wobei

- m 0, 1, 2
- X Cl, OR, R
- R gleiche oder verschiedene Alkylreste mit 1—8 C-Atomen, Benzyl-, Phenylethyl-, Cyclohexyl-, Cyclopentyl-

in Mengen von 0,01—5 Gew.-%, bei Temperaturen von 0-80°C dimerisiert, und das so gebildete 1,3-Diazacyclobutandion-2,4 nach einem Umsatz von 5—70% ohne vorgehende Desaktivierung des Katalysators aus dem Reaktionsgemisch durch Dünnschichtdestillation als Rückstand sowie Katalysator und Monomeres als Destillat isoliert. Die Herstellung der P-Verbindungen wird in Houben-Weyl, Methoden der Organischen Chemie, Bd. V, Seite 108 beschrieben.

Das mit dem erfindungsgemäßen Verfahren aus IPDI hergestellte Uretdion kann Oligomere, d. h. bis zu vier Uretdionringe enthalten, ist aber in jedem Fall zu mindestens bzw. mehr als 98% in IPDI rückspaltbar.

Die Durchführung des erfindungsgemäßen Verfahrens erfolgt in zwei Stufen, wobei

a) in der 1. Stufe mit Hilfe des beschriebenen Katalysators bis zu einem Umsatz, der die Förderung des Reaktionsgemisches im flüssigen Zustand (bei Raumtemperatur) noch zuläßt (ca. 40—60% IPDI-Umsatz), dimerisiert wird und
b) in einem 2. Schritt das nicht umgesetzte IPDI mit dem Katalysator durch Dünnschichtdestillation vom Reaktionsprodukt abgetrennt wird.

Das abdestillierte IPDI (plus Katalysator) kann wieder zur Reaktion eingesetzt werden.

Die Reaktionstemperatur liegt in einem Bereich von 0—80°C, vorzugsweise 10—30°C. Bei höheren Temperaturen macht sich deutlich die katalytische Umwandlung des Uretdions des IPDI in das entsprechende Isocyanurat bemerkbar.

Die erforderliche Katalysatormenge hängt sehr von der Art des eingesetzten Katalysators und der angewandten Temperatur ab. Mengen von 0,05—5 Gew.-% bezogen auf das eingesetzte IPDI sind im allgemeinen ausreichend. Bevorzugt finden 0,5—2 Gew.-% Phosphorigsäuretriamide Anwendung. Die Reaktionszeit, die Zeit in der z. B. 40—60 Gew.-% IPDI umgesetzt sind, hängt (bei konstanter Temperatur) in hohem Maße von der Konzentration sowie von der Art des eingesetzten Katalysators ab. Sie beträgt in der Regel 8—60 h. Die Reaktion kann in polaren Lösungsmitteln wie Estern, Chlorkohlenwasserstoffen, Ethern und Ketonen, oder lösungsmittelfrei durchgeführt werden. Bevorzugt arbeitet man lösungsmittelfrei.

Die Aufarbeitung des Reaktionsgemisches erfolgt, wie bereits angeführt, durch Dünnschichtdestillation bei 125—150°C und 0,0133—0,66 mbar.

Das erfindungsgemäß hergestellte reine Uretdion des IPDI ist bei Raumtemperatur hoch viskos (> $10^6$ mPa·s; bei 60°C: 13 000 mPa·s; bei 80°C: 1100 mPa·s). Sein NCO-Gehalt liegt im Bereich von 16,8—18%; d. h., daß mehr oder minder hohe Anteile oligomerer Uretdione des IPDI im Reaktionsprodukt vorliegen müssen. Der Monomergehalt liegt bei <1%. Der NCO-Gehalt des Reaktionsprodukts nach dem Erhitzen auf 180—200°C beträgt 37,1—37,7 Gew.-% NCO.

Das IR-Spektrum des Uretdions des IPDI zeigt neben einer intensiven Bande bei 2260 $cm^{-1}$ (N=C=O-Valenzschwingung) eine ebenfalls sehr intensive breite Bande bei 1760 $cm^{-1}$, die der C=O-Valenzschwingung des Uretdionrings zuzuordnen ist. Die für den Isocyanuratring charakteristische Bande bei 1690 $cm^{-1}$ tritt nicht auf.

Das Uretdion des IPDI soll als Zwischenprodukt zur Herstellung von Kunststoffen, Lacken und Schaumstoffen Verwendung finden. Es ist deshalb besonders wertvoll, weil es seines niedrigen Dampfdruckes wegen physiologisch ungefährlich ist und weil es gestattet, die im Uretdionring festgelegten Isocyanatgruppen durch Anwendung höherer Temperaturen wieder freizusetzen.

In besonderem Maße eignet es sich zur Herstellung von lösungsmittelhaltigen sowie lösungsmittelarmen Ein- und Zweikomponenten-Lacken, wie z. B. Coil Coating- und High Solid-Lacken, ferner für Polyurethan-Pulverlacke sowie für lösungsmittelfreie Ein- und Zweikomponenten-Beschichtungen.

## Beispiel 1

200 kg Isophorondiisocyanat und 2 kg Tris-(dimethylamino)-phosphin wurden in einem Reaktor bei Raumtemperatur 24 h zur Reaktion gebracht. Nach dieser Zeit betrug der NCO-Gehalt des Reaktionsgemisches ca. 30%. Ohne vorherige Desaktivierung des Katalysators wurde dieses Gemisch im Dünnschichtverdampfer aufgearbeitet. Das abdestillierte Isophorondiisocyanat, das nahezu quantitativ den Katalysator enthielt, wurde mit Frisch-Isophorondiisocyanat auf 200 kg ergänzt und wieder bis zu einem NCO-Gehalt von 30% stehen gelassen und anschließend wieder der Dünnschichtverdampfung zugeführt.

Für die Abtrennung des nicht umgesetzten Isophorondiisocyanats vom Isophorondiisocyanat-Uretdion stand eine Anlage mit zwei hintereinander geschalteten Dünnschichtverdampfern zur Verfügung. Folgende Bedingungen haben sich für die Isophorondiisocyanat-Abtrennung als optimal erwiesen.

| | Verdampfer I | Verdampfer II |
|---|---|---|
| Zulauf | 33 l/h · $m^2$ Aust.fl. | |
| Heißöl-Eintritt | 154°C | 156°C |
| Heißöl-Austritt | 150°C | 155°C |
| Vakuum | 7,7 mbar | 0,039 mbar |
| Destillat (% d. Zulaufs) | ca. 34% | ca. 35% |
| Reaktionsprodukt | | |
| NCO-Gehalt | 17,5% | |
| NCO-Gehalt n. Erhitzen auf 180°C, 1/2 h | 37,5%*) | |
| Viskosität | | |
| bei Raumtemperatur | > $10^6$ mPa·s | |
| bei 40°C | 125 · $10^3$ mPa·s | |
| bei 60°C | 13 · $10^3$ mPa·s | |
| Farbe | farblos bis leicht gelblich | |

*) Isophorondiisocyanat-Uretdion-Gehalt > 99%

### Beispiel 2

1000 Gew.-T. Isophorondiisocyanat und 20 Gew.-T. Tris-(dimethylamino)-phosphin wurden 10 h bei Raumtemperatur stehen gelassen. In dieser Zeit sank der NCO-Gehalt von 37,8% (reines Isophorondiisocyanat) auf ca. 31%. Ohne vorherige Desaktivierung des Katalysators wurde das Reaktionsgemisch bei 140° C/0,133 mbar im Dünnschichtverdampfer destilliert. Der Rückstand hatte einen NCO-Gehalt von 17,1%; beim Erhitzen auf 180° C ($^1/_2$ h) wurde ein NCO-Gehalt von 37% gefunden.

### Beispiel 3

1000 Gew.-T. Isophorondiisocyanat und 20 Gew.-T. Phosphorigsäure-(bisdimethylamid)-chlorid wurden gemischt und 30 h bei Raumtemperatur stehen gelassen. Nach dieser Zeit betrug der NCO-Gehalt des Reaktionsgemisches 32,5% NCO. Die Aufarbeitung erfolgte wie im Beispiel 2. Der Rückstand enthielt 16,8% NCO; beim Erhitzen auf 180° C ($^1/_2$ h) wurde ein NCO-Gehalt von 37,2% gefunden.

### Beispiel 4

1000 Gew.-T. Isophorondiisocyanat und 20 Gew.-T. Phosphorigsäuremethylester-bis-(diethylamid) wurden gemischt und 40 h bei Raumtemperatur stehen gelassen. Nach dieser Zeit betrug der NCO-Gehalt des Reaktionsgemisches 33,2%. Die Aufarbeitung erfolgte wie im Beispiel 2. Der Rückstand enthielt 17,0% NCO; beim Erhitzen auf 180° C ($^1/_2$ h) wurde ein NCO-Gehalt von 37,7% NCO gefunden.

### Beispiel 5
### (Vergleichsbeispiel)

1000 Gew.-T. Isophorondiisocyanat und 10 Gew.-T. Tributylphosphin wurden in einem Reaktor bei Raumtemperatur stehen gelassen. Da keine merkbare Reaktionswärme abgeführt werden mußte, war Rühren nicht erforderlich. Nach 40 h betrug der NCO-Gehalt der Reaktionsmischung ca. 30% NCO (= 45% Isophorondiisocyanat-Umsatz). Das Reaktionsgemisch, das noch niedrig viskos war, wurde ohne vorherige Desaktivierung des Katalysators der Dünnschichtdestillation zugeführt. Isophorondiisocyanat wurde bei 135° C/0,133 mbar vom Reaktionsprodukt abdestilliert. Der Rückstand hatte einen NCO-Gehalt von 17,4% NCO. Beim Erhitzen des Rückstands auf 180° C ($^1/_2$ h) wurde ein NCO-Gehalt von 33,5% NCO gefunden, d. h., daß das Reaktionsprodukt aus ca. 20 Gew.-T. trimerem Isophorondiisocyanat und aus ca. 80 Gew.-T. in der Hitze spaltbarem Isophorondiisocyanat bestand.

## Patentansprüche

1. Verfahren zur Herstellung eines praktisch isocyanuratfreien Uretdions aus Isophorondiisocyanat, welches in der Hitze zu über 98% wieder in Isophorondiisocyanat rückspaltbar ist, dadurch gekennzeichnet, daß man Isophorondiisocyanat, gegebenenfalls in einem inerten organischen Lösungsmittel, mit Hilfe eines Katalysators der allgemeinen Formel

$$X_mP(NR_2)_{3-m}$$

wobei

m 0, 1, 2
X Cl, OR, R
R gleiche oder verschiedene Alkylreste mit 1—8 C-Atomen, Benzyl-, Phenylethyl-, Cyclohexyl-, Cyclopentyl-,

in Mengen von 0,01—5 Gew.-%, bei Temperaturen von 0—80°C dimerisiert, und das so gebildete 1,3-Diazacyclobutandion-2,4 nach einem Umsatz von 5—70% ohne vorgehende Desaktivierung des Katalysators aus dem Reaktionsgemisch durch Dünnschichtdestillation als Rückstand sowie Katalysator und Monomeres als Destillat isoliert.

2. Verfahren nach Anspruch 1, dadurch gekennzeichnet, daß man als Katalysator die Verbindung

$$P[N(CH_3)_2]_3$$

verwendet.

3. Verfahren nach Anspruch 1, dadurch gekennzeichnet, daß man das aus dem Reaktionsgemisch zurückgewonnene nicht umgesetzte Isophorondiisocyanat wieder in die Reaktion einsetzt.

4. Verfahren nach Anspruch 1, dadurch gekennzeichnet, daß man den Katalysator in Mengen von 0,5—2 Gew.-% bei Temperaturen von 10—30°C einsetzt und das so gebildete 1,3-Diazacyclobutandion-2,4 nach einem Umsatz von 20—50% isoliert.

**Claims**

1. Process for the preparation of a uretdione, virtually free of isocyanurate, from isophorone diisocyanate, which uretdione can be re-disassociated under the action of heat into isophorone diisocyanate to an extent greater than 98%, characterised in that isophorone diisocyanate, if appropriate in an inert organic solvent, is dimerised at temperatures of 0—80°C with the aid of a catalyst of the general formula

$$X_mP(NR_2)_{3-m}$$

wherein

m 0, 1 or 2,
X is Cl, OR or R and
R represents identical or different alkyl radicals having 1—8 C atoms, benzyl, phenylethyl, cyclohexyl or cyclopentyl,

in quantities of 0.01—5% by weight, and the 1,3-diazacyclobutane-2,4-dione thus formed is, after conversion of 5—70%, isolated as the residue from the reaction mixture by thin-layer distillation, without previous deactivation of the catalyst, and the catalyst and monomer are isolated as the distillate.

2. Process according to claim 1, characterised in that the compound

$$P[N(CH_3)_2]_3$$

is used as the catalyst.

3. Process according to claim 1, characterised in that the unconverted isophorone diisocyanate recovered from the reaction mixture is recycled into the reaction.

4. Process according to claim 1, characterised in that the catalyst is used in quantities of 0.5—2% by weight at temperatures of 10—30°C and the 1,3-diazacyclobutane-2,4-dione thus formed is isolated after conversion of 20—50%.

**Revendications**

1. Procédé d'obtention à partir du diisocyanate d'isophorone d'un uretdione pratiquement exempt d'isocyanurate, pouvant se rediviser en diisocyanate d'isophorone, à plus de 98%, par chauffage, produit caractérisé en ce que le diisocyanate d'isophorone est dimérisé, éventuellement dans un solvant organique inerte, à l'aide d'un catalyseur de formule générale:

$$X_mP(NR_2)_{3-m}$$

où

m 0, 1, 2
X Cl, OR, R
R des restes alcoyle semblables ou différents, avec 1 à 8 atomes de carbone, desrestes benzyle, phényléthyle, cyclohexyle, cyclopentyle,

en proportions de 0,01 à 5% en poids à des températures de 0 à 80°C, et que le 1,3-diazocyclobutan-dione-2,4 ainsi formé, après une transformation de 5—70%, est isolé, sans désactivation préalable du catalyseur, à partir du mélange réactionnel, par distillation en couche mince, sous forme de résidu, le catalyseur et le monomère l'étant sous forme de distillat.

2. Procédé selon la revendication 1, caractérisé en ce que, comme catalyseur, on utilise le composé

$$P[N(CH_3)_2]_3$$

3. Procédé selon la revendication 1 caractérisé en ce que le diisocyanate d'isophorone qui n'a pas réagi, récupéré du mélange réactionnel, est remis en oeuvre dans la réaction.

4. Procédé selon la revendication 1, caractérisé en ce que le catalyseur est mis en oeuvre en proportion de 0,5 à 2% en poids à une température de 10 à 30° C, et que le 1,3-diazocyclobutandione-2,4 ainsi formé est isolé avec un rendement de 20—50%.